# EUROPEAN PATENT APPLICATION

(11) **EP 4 219 748 A1**
(43) Date of publication of application: **02.08.2023**
(21) Application number: 23164311.5
(22) Date of filing: 21.08.2020
(51) Int. Cl.: C12Q 1/6806, B01L 3/00

(54) **ISOLATION OF HIGH MOLECULAR WEIGHT DNA USING BEADS**

(30) Priority: 21.08.2019 US 201962889753 P; 22.08.2019 US 201916547844
(62) Divisional of application: 20192200.2
(71) Applicant: New England Biolabs, Inc., Ipswich, MA 01938 (US)
(72) Inventor: KOETSIER, Paul A., Ipswich, 01938-2723 (US); TARON, Barbara W., Ipswich, 01938-2723 (US); CANTOR, Eric J., Ipswich, 01938-2723 (US)
(74) Representative: Griffin, Philippa Jane

(57) **Abstract**

Provided herein are kits, compositions and methods for isolating high molecular weight (HMW) DNA having a desired length in a rapid reproducible manner using beads of at least 200 µm to adsorb DNA from a cell lysate. A device is described for retaining the beads while permitting washing of the DNA bound to the beads and eluting the DNA from the beads. The device permits the DNA eluant released from the beads to exit the device without shearing. The device also permits recycling of the beads.

## Description

### BACKGROUND

Several methods exist to analyze long nucleic acid molecules. For example, nanopore sequencing is routinely used to sequence DNA molecules that are tens to hundreds of kilobases in length (see for example, Oxford Nanopore Technologies, Oxford UK). Read lengths in excess of two megabases (Mb) have been reported from one nanopore sequencing study (Payne, et al, Bioinformatics. 2019 35: 2193-2198). Likewise, the PacBio platform (Pacific Bioscience, Melo Park, CA) is capable of sequencing nucleic acid molecules that are tens of kilobases in length. Other genome mapping platforms such as the nanochannel array-based method (described by Lam, et al Nat. Biotechnology 2012 30: 771-776, as developed by BioNano Genomics, San Diego, CA) also provide ways to analyze long nucleic acid molecules.

Long range genome analysis methods such as those described above require purified high molecular weight (HMW) DNA as an input. HMW genomic DNA (gDNA) has traditionally been purified from cells by phenol/chloroform extraction and subsequent alcohol precipitation while spooling the gDNA around a glass rod.

There are several methods for purifying HMW gDNA from small volume samples. These include magnetic discs, magnetic beads and silica filters. However, each of the existing methods have certain deficiencies. Some of the methods result in shearing of the HMW DNA. Other methods require multiple handling steps and therefore are not ideally suited to handling large numbers of samples at the same time. As the methods for HMW genome analysis improve and the number of clinical, diagnostic and research samples increase, there is a continued need to improve the reliability and efficiency of HMW DNA purification.

### SUMMARY

Provided herein, among other things, is a method that utilizes a composition comprising: (i) one or more beads, for example, glass beads, for example, 1-50 beads, where each bead has a smallest dimension of at least 200 micrometers (µm), preferably greater than 200 µm, for example, at least 1 mm, for example, in the range of 1 mm-6 mm (e.g. about 4 mm), (ii) a DNA preparation comprising HMW DNA, for example, having a median size of at least 35 kilobases (kb), such as at least 50 kb; and (iii) a DNA precipitant. In some embodiments, the one or more beads is substantially spherical, and the smallest dimension corresponds to the diameter of the bead. In some embodiments, the bead sizes may be variable in a single kit or composition for use in the bead retaining tube, although the use of beads of the same or closely similar size may be preferable in any single bead retaining tube. The method may involve at least one of the following steps: incubating the composition to adhere the DNA to the one or more beads; removing unbound material from the one or more beads; washing the one or more beads with a wash buffer; releasing the DNA from the one or more beads into an elution buffer; and separating the released DNA in the eluate from the one or more beads.

The DNA preparation may be a pre-purified HMW DNA or a complex mixture that includes HMW DNA and macromolecules, for example, a cell lysate. The DNA preparation may result from lysing cells to produce a lysate, centrifuging the lysate, and using the supernatant as the DNA preparation. Alternatively, the DNA preparation may result from lysing cells to produce a lysate and using the lysate as the DNA preparation . Alternatively, the DNA preparation may be a lysate that is produced by agitating cells in a lysis buffer at an agitation rate in the range of 300 rpm -2000 rpm. Agitation may occur at a temperature of least 37°C -56°C. The speed of agitation determines the median length of the DNA used in the DNA preparation. The DNA preparation may be, for example, a DNA solution.

The DNA precipitant used in the method may include a chaotropic salt, a crowding reagent such as Polyethylene glycol (PEG), and/or an alcohol such as ethanol or isopropanol. The chaotropic salt and/or crowding agent may be present in the DNA precipitant at high concentration.

Removal of unbound material may be achieved by pouring or pipetting wherein pipetting utilizes for example, a wide bore pipette tip. A washing step may be included that uses an alcohol-containing wash buffer. The washing step can remove residual material from the DNA adhered to the beads. The washing step may be done by washing beads in an alcohol-containing wash buffer, pouring beads into bead retaining tube, and spinning the bead retaining tube to remove the supernatant leaving behind DNA adhered to the beads.

The DNA can be released from the beads into an eluate by washing the DNA-beads with a suitable elution buffer. The DNA beads and elution buffer may be combined in a bead retaining tube that can be centrifuged to permit the eluate to be collected in a collection tube or outer tube while the beads remain in the bead retaining tube. In one example, the bead retaining tube is inserted into the collection tube prior to centrifugation. In this configuration, the bead retaining tube has one or a plurality of exit openings where the openings have a size (in at least one dimension e.g. diameter) that is smaller than the smallest dimension of the one or more beads. The one or a plurality of exit openings may be substantially circular in shape and has a diameter less than smallest dimension of the one or more beads. The one more exit opening(s) preferably has a size (e.g. diameter) of at least 0.5 mm. The bead retaining tube is designed so that the one or more beads do not block the one or more exit openings so as to allow the flow of the bead containing eluate out of the bead containing tube into the collection tube. This may be achieved by the bead retaining tube comprising one or more ridges, flanges, or collars that support the one or more beads within the tube above the one or more exit openings. In one embodiment, the exit opening is in the form of a sieve or porous mesh, comprising a series of exit openings arranged in a grid pattern. Alternatively, the beads and DNA elution buffer may be contained in a closed tube and the DNA containing elution buffer removed by pouring or pipetting the supernatant into a collection tube. The released DNA is a high molecular weight (HMW) DNA has, for example, a median size of at least 25 kb, such as at least 35 kb - 50 kb.

Also provided is a composition comprising: one or more beads (such as glass beads) having a smallest dimension of at least 200 µm; a DNA preparation comprising HMW DNA that has for example, a median size of at least 35 kb or at least 50 kb; and a DNA precipitant, wherein the precipitant may comprise an alcohol, and/or chaotropic salts or a crowding reagent at high concentrations commonly used in the art. In some embodiments, the one or more beads have a smallest dimension of at least 200 µm, such as at least 1 mm, such as in the range of 1 mm to 6 mm (e.g. about 4 mm). The one or more beads may, for example, be substantially spherical and have a diameter of at least 200 µm, such as at least 1 mm, such as in the range of 1 mm - 6 mm (e.g. about 4 mm). In some embodiments, the one or more beads is 1-50 beads, such as 1-50 spherical glass beads. The DNA preparation may be obtained from a cell lysate or may be a cell lysate or tissue lysate, or from HMW DNA previously purified with other methods. The DNA preparation may be a DNA solution. Where the DNA precipitant is an alcohol, the alcohol may be ethanol or isopropanol. The composition, comprising the one or more beads, the DNA preparation, and the DNA precipitant, may be contained in a bead retaining tube, wherein the DNA adheres to the beads.

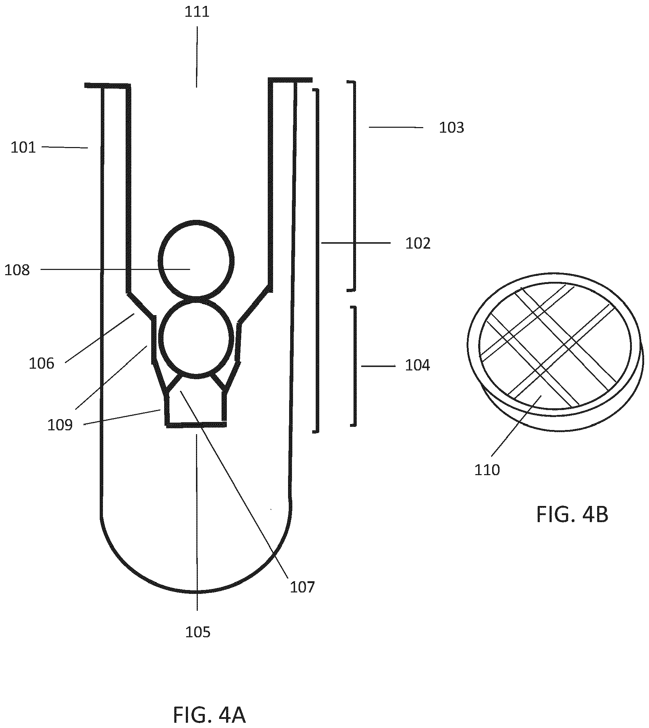

Also provided is a kit comprising: (a) one or more beads (for example glass beads) that have a smallest dimension of at least 200 µm (e.g. at least 500 µm or at least 1mm); and (b) a bead retaining tube having an opening for receiving a DNA preparation (e.g. DNA solution) and one or more outlet openings, wherein each outlet opening has a size in at least one dimension e.g. diameter) that is smaller than the smallest dimension of each of the beads. The outlet opening preferably has a size of at least 500 µm (0.5 mm) for beads having a smallest dimension of greater than 500 µm. The bead retaining tube is designed so that the one or more outlet openings are not blocked by the one or more beads. This may be achieved by having one or more ridges, flanges, or collars around the outlet opening on which the beads may sit as fluid flows around the beads and then exits the tube as illustrated in Fig 4A. In some kits, the one or more beads having a smallest dimension of at least 200 µm, are beads that have a smallest dimension of at least 1mm, such as in the range of 1 mm to 6 mm (e.g. about 4 mm). Some kits may further comprise an outer collection tube, wherein the bead retaining tube fits into the collection tube. The beads for use in the kit may be formed of a material that has a charged surface and that is preferably smooth although the surface may be rough as long as this does not damage the adsorbed DNA. In one example, the beads in the kit are made from glass or ceramic material. The beads may have any desired shape. In one example, the one or more beads are substantially spherical, and have a diameter of at least 200 µm, optionally at least 1mm, such as in the range of 1 mm - 6 mm (e.g. about 4 mm). For example, the beads may be one or more spherical, glass or ceramic, beads. The one or more beads may be 1-50 beads, such as where 1-50 beads of 1 mm-6 mm in size (e.g. 1-50 spherical beads of 1 mm-6 mm in diameter) may be included with each bead retaining tube. The number of beads that are optimal is inversely proportional to the size of the beads, where more beads are required at the lower end of the described size range. The bead retaining tube has a volume large enough to contain the beads. The one or more exit openings of the bead retaining tube may have a size (in at least one dimension e.g. diameter) of at least 0.5 mm, so long as this is less than the size of the smallest dimension of the one or more beads. Thus, if the one or more beads each have a smallest dimension of at least 1mm (e.g. 1-6 mm) the one or more exit openings have a size that is at least 0.5 mm but less than 1mm. In one embodiment, the one or more exit openings of the bead retaining tube are circular and have a diameter that is smaller than the smallest dimension (e.g. diameter) of the beads; such as wherein the one or more beads are spherical and each have a diameter of at least 1mm and the one or more circular exit openings have a diameter that is at least 0.5 mm but less than 1mm.

The outer tube can be used for collecting eluate containing DNA. Where an outer collection tube is provided, the bead retaining tube may fit into the outer tube. The collection tube may for example have a volume of 1 milliliter (ml) - 2 ml.

The kit may further comprise an elution buffer, an alcohol-containing wash buffer, a lysis buffer and/or enzymes (such as Proteinase K and RNase A) for lysing and/or purifying DNA in a DNA preparation; where the reagents and buffers described above may be provided in the same or different tubes or various combinations of tubes containing individual reagents and/or buffers and tubes containing mixtures of reagents and/or buffers.

The above described method uses in at least one example, the compositions that are summarized above and may optionally use the kits as summarized above, wherein the methods, compositions and kits are described in detail below.

### BRIEF DESCRIPTION OF THE FIGURES

The figures and drawings are intended to illustrate one or more versions of the compositions and/or methods described herein. Unless stated otherwise, these are not intended to be limiting for the purpose of interpreting the scope of any claims.

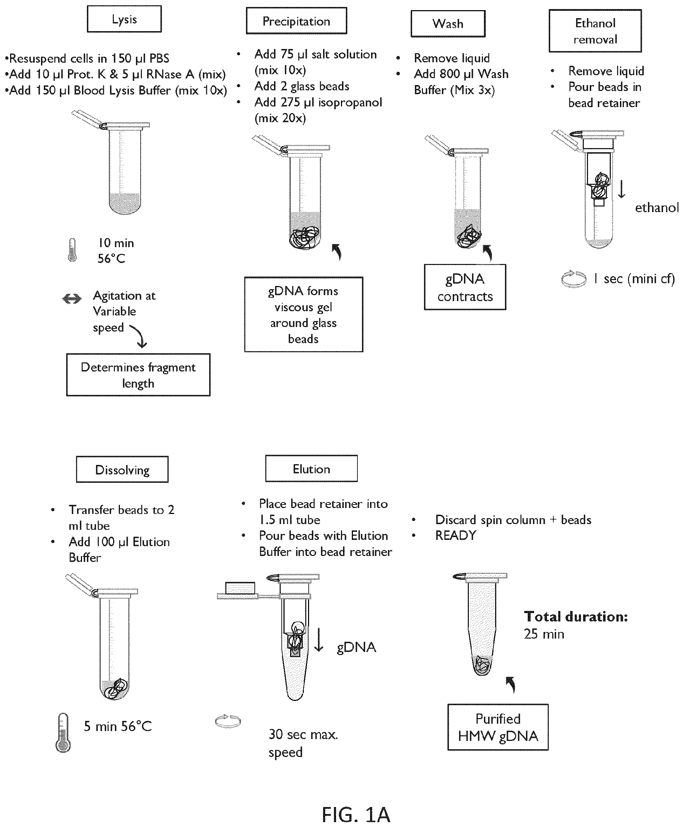

FIG. 1A schematically illustrates an example of how HMW gDNA can be isolated and purified from mammalian cells using the present method.
   Lysis: Resuspend cells in 150 µl PBS. Add 10 µl Proteinase K & 5 µl RNase A (mix). Add 150 µl Blood Lysis Buffer (mix 10X), 10 minutes, 56°C. Agitation at variable speed determines fragment length.
   Precipitation: Add 75 µl salt solution (mix 10x). Add 2 glass beads. Add 275 µl isopropanol (mix 20x) gDNA forms viscous gel around glass beads.
   Wash: Remove liquid. Add 800 µl wash buffer (mix 3X). gDNA contracts.
   Ethanol removal: Remove liquid, pour beads in bead retainer, 1 second mini centrifugation.
   Dissolving: Transfer beads to 2 ml tube, add 100 µl elution buffer incubate 5 minutes at 56°C.
   Elution: Place bead retainer into 1.5 ml collection tube. Pour beads with Elution Buffer into bead retainer, centrifuge for 30 seconds max.
   Collect purified HMW gDNA.
   Total time =25 minutes.

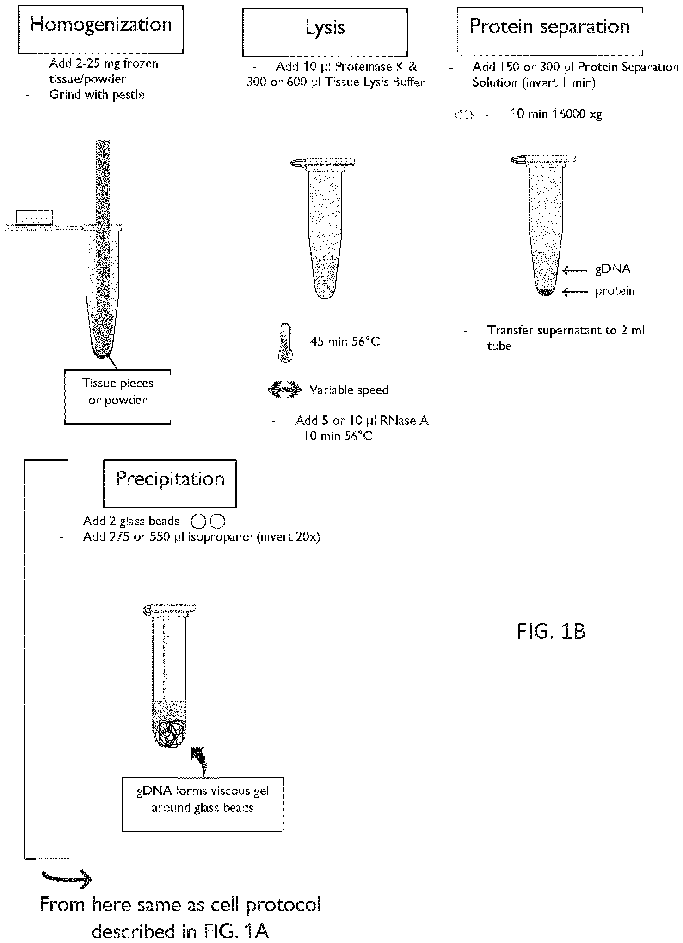

FIG. 1B schematically illustrates an example of how HMW gDNA can be isolated and purified from tissue pieces or powder.
   Homogenization: Add 2 mg-25 mg frozen tissue/powder, grind with pestle.
   Lysis: Add 10 µl Proteinase K and 300 or 600 µl tissue lysis buffer. Heat 56°C for 45 minutes, select agitation speed. Add 5 µl or 10 µl RNase A and incubate 10 minutes at 56°C.
   Protein separation: Add 150 µl or 300 µl protein separation solution (invert 1 minute) centrifuge 10 minutes at 16000 g. Transfer supernatant to 2 ml tube.
   Precipitation: Add 2 glass beads and 275 µl -550 µl isopropanol (invert 20X). gDNA forms viscous gel adsorbed to glass beads.
FIG. 2A - FIG. 2B show examples of bead retaining tubes and collection tubes, where the bead retaining tubes have at least one exit opening.
FIG. 2A illustrates flat bottomed bead retaining tubes that could be used in the present method and separately the collection tube suitable in design for all steps of the method.
FIG. 2B illustrates 2 ml bead retaining tubes that have one collar that could be used in the present method and separately the collection tube suitable in design for all steps of the method.
FIG. 3A - FIG. 3B illustrates examples of 2 ml bead retaining tubes, which contain beads having a 4 mm diameter.

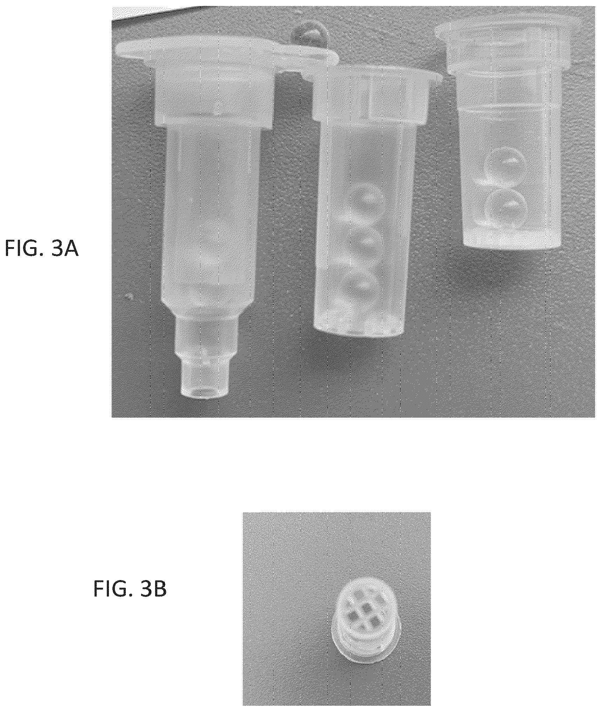

FIG. 3A shows a sideways view of two types of 2 ml bead retaining tubes, one having two collars, which contain beads having a 4 mm diameter, and the second bead retaining tube having a flat-bottom with a plurality of openings containing the 4 mm beads.
FIG. 3B shows an end-on view of a flat-bottomed tube in FIG. 3A showing the plurality of openings.
FIG. 4A -FIG. 4B are diagrammatic representations of parts of the bead retaining tubes shown in FIG. 3A-FIG. 3B.
FIG. 4A shows the two collared bead retaining tube in FIG. 3A contained in a collection tube. The bead retaining tube (102) is contained within a collection tube (101). The body of the bead retaining tube is (103). Two collars (104 and 109) of decreasing size are shown where the exit opening (105) is greater than 0.5 mm in diameter. The sloping sides of the tubes (106) connect provide a transition to a narrower collar. 2 ridges are shown with one being marked (107) on which one or a plurality of beads (108) sit to permit DNA in the eluant to flow into the collection tube and avoid blocking the exit opening. An opening at the top of the bead-retaining tube (111) is oppositely placed with respect to the exit opening at the bottom of the tube (105), where the opening is used for accepting the DNA preparation and the beads.
FIG. 4B shows the flat exit opening containing a plurality of exit holes each being at least 0.5 mm in diameter (110) at the base of a bead collection tube shown in FIG. 3A and FIG. 3B.

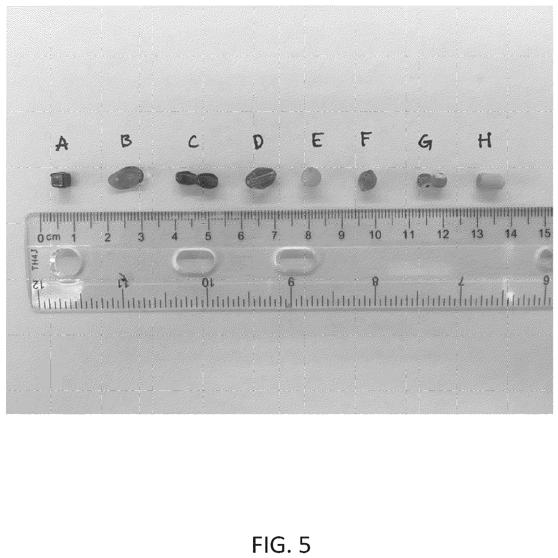

FIG. 5 shows that shape is not critical for beads where 8 different sized jewelry glass beads (A-H), were used and any shaped bead of appropriate format can serve as binding matrix. Results are shown in Table 4.

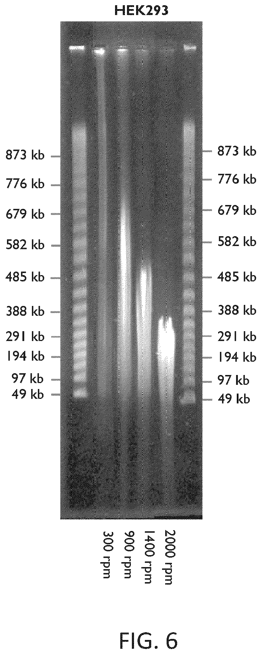

FIG. 6 shows the size ranges of gDNA isolated from human embryonic kidney 293 (HEK293) cells at various agitation speeds during the lysis protocol prior to purification. HMW gDNA from 1×10⁶ HEK293 cells was isolated according to the protocol for cultured mammalian cells as described in Example 1. During lysis samples were agitated at different agitation speeds (300 rpm, 900 rpm, 1400 rpm and 2000 rpm). 300 nanograms (ng) of each sample was loaded on a 0.75% pulsed field agarose gel using 0.5x KBB buffer and a Pippin Pulse^{™} electrophoresis power supply (Sage Science, Inc, Beverly MA). A molecular weight marker the Lambda PFG Ladder (New England Biolabs, Ipswich, MA) was used. An agitation speed of 300 rpm resulted in largest gDNA and an agitation speed of 2000 rpm resulted in DNA that was no larger than 300 kb.

### DETAILED DESCRIPTION OF THE EMBODIMENTS

Unless defined otherwise, all technical and scientific terms used herein have the same meaning as commonly understood by one of ordinary skill in the pertinent art to which this disclosure belongs. Still, certain terms are defined herein with respect to embodiments of the disclosure and for the sake of clarity and ease of reference. Sources of commonly understood terms and symbols may include: standard treatises and texts such as Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); Singleton, et al., Dictionary of Microbiology and Molecular biology, 2d ed., John Wiley and Sons, New York (1994), and Hale & Markham, the Harper Collins Dictionary of Biology, Harper Perennial, N.Y. (1991) and the like.

As used herein and in the appended claims, the singular forms "a", "an", and "the" include plural referents unless the context clearly dictates otherwise. For example, the term "a protein" refers to one or more proteins, i.e., a single protein and multiple proteins. The claims can be drafted to exclude any optional element when exclusive terminology is used such as "solely," "only" are used in connection with the recitation of claim elements or when a negative limitation is specified.

Aspects of the present disclosure can be further understood in light of the embodiments, section headings, figures, descriptions and examples, none of which should be construed as limiting the entire scope of the present disclosure in any way. Accordingly, the claims set forth below should be construed in view of the full breadth and spirit of the disclosure. Each of the individual embodiments described and illustrated herein has discrete components and features which may be readily separated from or combined with the features of any of the other several embodiments without departing from the scope or spirit of the present teachings. Any recited method can be carried out in the order of events recited or in any other order which is logically possible.

Numeric ranges are inclusive of the numbers defining the range. All numbers should be understood to encompass the midpoint of the integer above and below the integer i.e. the number 2 encompasses 1.5-2.5. The number 2.5 encompasses 2.45-2.55 etc. When sample numerical values are provided, each alone may represent an intermediate value in a range of values and together may represent the extremes of a range unless specified.

Provided herein are examples of a method for isolating HMW DNA. Advantages of the method include at least one of the following:
(a) Rapid process for obtaining purified HMW DNA from cells or tissues within 30 minutes-90 minutes from beginning to end of the workflow;
(b) Reproducibility of the method for producing a high yield of pure HMW DNA as determined by 260/280 and 260/230 wavelength readings.
(c) Ability to scale input amounts and still obtain a high yield of HMW DNA (see Table 1A);

**Table 1A**

| Number of Cells | Typical Yield (µg) |
|---|---|
| 1x10⁵ | 0.5-1.0 |
| 5x10⁵ | 5.5-6.5 |
| 1x10⁶ | 11.5-13 |
| 5x10⁵ | 57.5-65 |
| 1x10⁷ | 110-125 |

(d) Tunability of DNA size. This can be achieved by selecting a suitable agitation speed. The higher the agitation, the smaller the DNA size. Agitation with 300 rpm -500 rpm can provide DNA in the Mb size range. Agitation at maximum speed (e.g. at about 2000 rpm) produces DNA that is 50 kb -250 kb which is optimal for sequencing on an Oxford Nanopore sequencing platform (Oxford UK);
(e) Versatility of the method for use with for any desired cell type or tissue. Examples of embodiments for obtaining HMW from bacteria, eukaryotic cells and tissues are provided below.

Examples of workflows are provided in FIG 1A-FIG. 1B. The workflows include one or more steps from the following:
Lysis of cells or tissue, and protein removal and release of gDNA having a tunable size;
Precipitation of DNA onto glass beads (e.g. using an alcohol such as ethanol or isopropanol);
Washing of glass beads with captured DNA;
Eluting the DNA from the glass beads with separation of the eluate from the glass beads to recover pure HMW DNA.

A feature of the present method is the use of beads that provide a suitable surface around which the HMW DNA can be wrapped and which are larger than 200 µM in size to avoid interbead shear of bead adsorbed DNA. The term "beads" as used herein refers to small objects or matrices for adsorbing macromolecules from a solution. Beads are most commonly spherical but can also be other shapes that are regular or irregular. Some examples of various bead shapes that were tested and found effective are provided in Table 4 and FIG. 5. Thus, the beads may be e.g. spherical, ovoid, disc shaped, cylindrical, cube, cuboid, hourglass/8-shaped, pyramid shaped etc. In one embodiment, the beads are spherical. Beads are most commonly smooth. The material of the beads is preferably glass for reasons of cost and ease of availability. However, any material can be used that has a charge, is polar, and provides a surface to which DNA adsorbs. The charged surface enables the DNA to adhere to the beads. Such materials include ceramics and clay. Moreover, although transparent glass beads are used in the examples, Table 4 and FIG. 5 provides data on colored glass that works effectively too. The size of the beads may vary according to the length of DNA intended for purification. Beads with a diameter of at least 1 mm are preferred for reasons that include that they can be manually handled by means of tweezers for placing the beads in the tubes. Beads less than 200 µm should be avoided. Below 200 µm, the beads can move against each other to cause undesirable shear damage to the DNA adsorbed to their surface.

When 2 ml tubes are used for sample processing, the total surface area of the beads per tube or sample may be in the range of 75 mm² to 150 mm² in some cases. For example, if a bead size of 4 mm diameter is selected, then two beads can be used per 2 ml tube/sample to provide a surface area in this range. In this example, the total surface area of the beads is 100.54 mm². If smaller beads are selected (e.g., beads that have a diameter in the range of 200 µm to 4 mm) then more beads should be used. The following table describes the relationships between diameter, surface area/bead, bead number to obtain a total bead surface area of approximately 100 mm² and weight of borosilicate beads (which have mass/volume of 2.23 milligram (mg)/mm³) to obtain the same.

**Table 1B**

| **Bead Diameter (mm)** | **Surface area/bead (mm2)** | **Number of beads** |
|---|---|---|
| 0.25 | 0.2 | 500 |
| 0.5 | 0.79 | 127 |
| 1 | 3.14 | 32 |
| 2 | 12.57 | 8 |
| 3 | 28.27 | 4 |
| 4 | 50.27 | 2 |
| 5 | 78.54 | 1 |
| 6 | 113.1 | 1 |

Where a method for purifying large molecules of DNA from a relatively small sample of a cell lysate or tissue biopsy is desired, beads in the 1 mm to 6 mm range may be used in a tube capable of holding about 1 ml-2 ml. In this example, the bead number is selected to provide a total surface area of at least about 100 mm². This may be achieved using up to 30-50 beads of 1 mm diameter. Larger number of beads may be used if the bead size is less than 1 mm in diameter as long as the bead diameter is greater than 200 µm. For example, to achieve a 100 mm surface area, one bead of 5 mm or 6 mm diameter may be used, two beads of 4 mm diameter may be used, four beads of 3 mm diameter may be used, eight beads of 2 mm diameter may be used, or 32 beads of 1 mm diameter may be used. Where a sample is in a volume greater than 2 ml, the size of the bead retaining tube, collection tube and the available surface area for a preferred size and number of beads can be scaled accordingly.

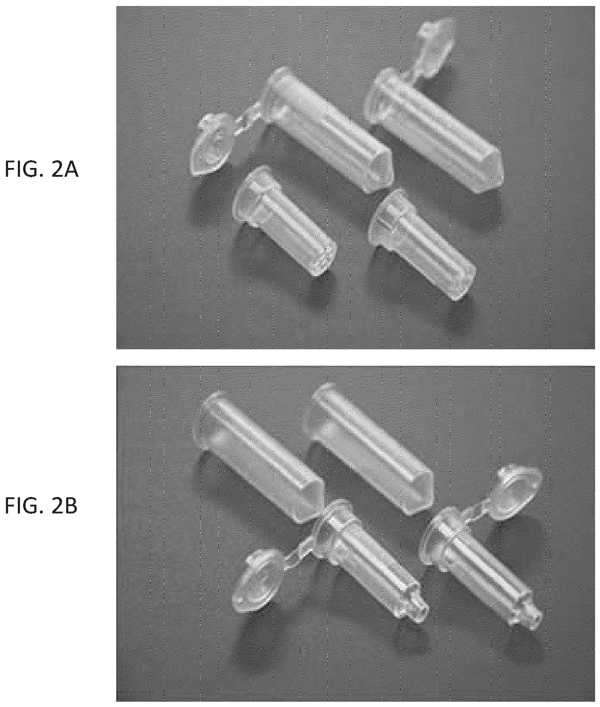

As described below, certain tubes used for various steps of the method may have some particular characteristics that are advantageous for various embodiments of the method. For example, microfuge tubes are preferable for small samples of about 2 ml or less, as these can be used in a microcentrifuge. In addition, an inner tube (here referred to as a bead retaining tube) containing at least one exit opening can be inserted into an outer tube (collection tube). The outer tube may be a microfuge tube (see for example, FIG. 2A-FIG. 2B).

Embodiments of the methods may include the steps of precipitating the DNA onto the beads, washing the beads, and then eluting the DNA off the beads. The physical separation of DNA from the beads can be achieved conveniently and effectively through the one or more exit openings in the base of the bead retaining tube where the elution and separation of DNA into a collection tube can be enhanced by centrifugation. The size of the exit opening should be less than the size of the beads to enable separation of DNA from the beads. The 'size' of the exit opening refers to the length of at least one dimension of the exit opening - e.g. width, length of at least one side, diagonal measurement, diameter. If the exit opening is substantially circular, the 'size' of the exit opening is the diameter of the exit opening. The one or more exit openings in the bead retaining tube are each preferably at least 0.5 mm in size (e.g. diameter) so as to avoid shearing of the HMW DNA passing through the exit openings. Where exit opening(s) is for example 0.5 mm in size (e.g. diameter), the bead(s) should have a smallest dimension (e.g. diameter, if spherical) that is at least slightly larger than 0.5 mm, such as at least about 1 mm; and where the exit opening(s) is for example 1 mm in size (e.g. diameter), the bead(s) should have a smallest dimension (e.g. diameter, if spherical) that is at least slightly larger than 1 mm (e.g. at least about 2mm to 6 mm).

Examples of two types of tubes with at least one exit opening are provided here for use in method steps.

The first example is a bead retaining tube (102) into which DNA containing beads (108) are placed through an opening (111), where the bead retaining tube has a collar (109) and an exit opening (105) as shown in FIG 4A. The bead retaining collar has a shape that is preferably not tapered but may be cylindrical or part cylindrical and optionally the wall of the cylindrical collar between the bead containing body of the bead and the exit opening may be partially cut away. One cylindrical collar is shown on the tubes in FIG. 2A and two collars are shown in FIG. 3A and FIG. 4A. In FIG. 3A and FIG. 4A, at least the ultimate bead retaining collar has a reduced diameter compared to the bead containing portion of the tube. The beads are prevented from entering the ultimate collar (109) with exit opening (105) because of size of the entry aperture being smaller than the bead diameter. Any number of collars having any desired shape that does not result in shearing of the DNA may be used. The collar may be replaced by any other bead retaining structure associated with the exit opening that permits eluted DNA to pass through without shear. In addition, it is a preferred feature of the bead retaining tube that the beads do not block the exit opening. This may be achieved here by a plurality of internal projecting ridges or spacers (107) that are positioned on or near the bead retaining collar where the number of ridges may for example include 3 or 4 protruding ridges that are evenly spaced and onto which the beads or a bead may sit providing space for DNA containing eluate to flow around. Other numbers of ridges and their distribution or other protruding structures for holding beads having a selected size preferably at least 1 mm may be selected allowing the DNA eluate to flow around the beads and enter the collection chamber through the exit opening. In this way DNA containing eluant can flow easily into a collection tube (101) by gravity or with the aid of a centrifugation step. The one or more exit openings of a bead retaining tube may have an internal spacer or ridge that spaces the beads from the exit opening during centrifugation. The interface between the collar and the bead containing body of the bead retaining tube (106) may be sloping for example at a 45° angle or curved so as to minimize shear as the DNA eluant passes into the collection tube.

In a second example of a bead retaining tube containing the beads, separation of HMW DNA from the beads may not utilize a collared exit funnel. Instead, the tube may have a flat bottom with a grid or plurality of exit openings (110) each opening having a size of at least 0.5 mm allowing the beads having a greater size to remain in the tube while the eluate containing DNA flows through without obstruction (see FIG. 3A-FIG. 3B). This contrasts with conventional use of the fine membranes and frits in silica-based DNA preparation methods that have pore sizes less than 0.5 mm and cause unwanted shearing of HMW DNA.

If beads having a minimum size of less than 0.55 mm but at least 200 µm are used for HMW purification, it is recommended that a batch method is used where a tube that does not have an exit opening is used for the elution step. After release of the DNA from the beads, the supernatant containing the DNA can be removed using a wide bore pipette tip.

The present method avoids many of the limitations of conventional methods of DNA purification for HMW DNA molecule purification. The present method does not require vortexing or that the DNA sample pass through any fine holes. Moreover, the method does not use beads having a diameter of less than 200 µm such as the commercially available magnetic beads, thereby eliminating many sources of shearing that are associated with other methods (see for example, Jakobi, et al., Anal Biochem., 175(1):196-201, 1988; US Patent No. 6,255,477; Nanaassy, et al. Anal Biochem., 365(2), 2007: 240-245; Steiner, et al., Nucleic Acid Research, 23:13, 2569-2570 1995; and Vogelstein, et al., PNAS, 76:2, 615-619, 1979). The median length of the isolated gDNA produced by the present method is at least 50%, at least 70% or preferably at least 90% of the median length of the input DNA. Thus, for example, if the median length of the input DNA is about 50 kb, the median length of the isolated gDNA produced by the method is at least about 25 kb, 35 kb, or 45 kb. The method can be used to purify HMW DNA from virtually any type of DNA sample, e.g., isolated white blood cells, cultured cells, cells from a biopsy, and bacterial cells, or previously purified HMW extracted with other methods, etc.

The gDNA isolated by the present method can be analyzed using any of a wide range of long-range analysis methods, including long read DNA sequencing and genotyping.

The term "long DNA" and "high molecular weight (HMW) DNA" refer to DNA that is generally larger than 30 kb, for example larger than 35kb, for example at least 50 kb, for example as much as 100 kb, 200 kb, 300 kb, 500 kb, or 1 Mb. Although the present methods are particularly suited to obtaining HMW DNA, the methods may be used for obtaining shorter DNA also.

The term "DNA precipitant" refers to: a high salt concentration of a chaotropic salt; an alcohol; and/or a crowding agent. While not wishing to be limited by theory, a chaotrope can be used to precipitate DNA because nucleic acids are covered by a shell of hydration consisting of water molecules that maintain the solubility of DNA in aqueous solutions. With the addition of chaotropic ions to the nucleic acid, this relatively ordered structure of water molecules of the hydrate shell is destroyed. The chaotropic salts create a hydrophobic environment for DNA precipitation. As a further feature of the chaotropic salts, the respective cations saturate the beads with positive charges, which still improves the binding of nucleic acids under hydrophobic conditions. Chaotropic salts increase the solubility of nonpolar substances in water. They denature proteins because they have the ability to disrupt hydrophobic interactions. They do not denature DNA or RNA. Examples of chaotropic salts used in DNA purification include guanidine chloride and guanidine thiocyanate. For example, Promega describes the use of high concentrations of chaotropic salts (e.g. guanidine chloride HCL) to cause DNA to selectively bind to silica membranes. Promega also offers a kit called Reliaprep^{™} (Promega Corporation, Madison, Wl) where the gDNA is bound to the membrane (in the case a cellulose material) with the help of a crowding reagent (PEG). This kit does not use chaotropic salts or alcohol as a precipitant. Chaotropic guanidinium thiocyanate (GuSCN) enhanced DNA-silica adsorption was described by Katevatis et al., 2017 PLOS ONE 12(5): e0176848. Other examples of DNA precipitants are provided by Gene Link, Elmsford, NY (DNA & RNA Precipitation Solutions). Adding ethanol decreases the dielectric constant of the solution. In some embodiments, the method may involve producing a composition comprising one or more beads (as described above) (e.g., 1-50 glass beads) that have a diameter of at least 200 µm, a DNA preparation (e.g. DNA solution) comprising DNA that has a median length of at least 50 kb, e.g., gDNA that has a median length of at least 50 kb, at least 200 kb, at least 500 kb or at least 1 Mb, and a DNA precipitant (e.g., a high concentration of chaotropic salts and/or ethanol or isopropanol, with or without salt, wherein the precipitant in the composition at a concentration and pH that will cause precipitation of the DNA). The precipitated DNA adheres to the surface of the one or more beads and may appear to form a loose gel that encapsulates each of the beads that becomes more tightly associated with the beads during the washing steps that follow precipitation.

After the DNA has been adhered to the beads, the unbound material (which remains in the liquid surrounding the beads) can be removed in way that avoids disturbing the one or more beads. This can be done by pipetting or aspiration, for example. Next, the one or more beads are washed in a wash buffer, e.g., an alcohol containing wash buffer such as 70% ethanol or the like. This step can be done by, for example, adding a volume of the wash buffer to the beads in a container, gently inverting the container, and then carefully removing the wash buffer without unduly disturbing the one or more beads. If necessary, any residual wash buffer can be removed by centrifugation. This may be done, for example, by decanting the beads and residual wash buffer into a bead retaining tube, and then centrifuging the beads while they are in the bead retaining tube. In these embodiments, the residual wash buffer should pass through a plurality of holes of preferably at least 0.5 mm (but smaller than the smallest dimension e.g. diameter of the beads) at the bottom of the column, leaving the washed beads that have a minimum size (e.g. diameter) of greater than 0.5mm (e.g. at least 1mm) in the bead retaining tube.

Next, the DNA may be released from the beads into an elution buffer. This may be done by transferring the beads to another container, adding a volume of elution buffer (e.g., a Tris/EDTA solution or other buffers known to release DNA (see for example, Katevatis, et al., (2017)) to the beads, and then incubating the beads, e.g., for 5 minutes -10 minutes at room temperature or at an elevated temperature (e.g., 37-56°C) to elute the DNA from the beads. Next the eluted DNA is separated from the one or more beads.

Transfer of DNA containing beads can be achieved by decanting the DNA containing beads into a bead retaining tube that is within an outer collection tube, adding elution buffer to release the DNA and centrifuging the beads while they are in the bead retaining tube. In these embodiments, the eluate passes through the exit opening or openings at the bottom of the bead retaining tube into the collection tube. The eluted DNA, which is now in the collection tube, can then be stored, further processed, or analyzed directly.

The initial DNA sample prior to purification may be purified gDNA in water or buffer, a complex mixture comprising DNA and biological macromolecules, a cell lysate, a tissue lysate, or a cell lysate that that has been subjected to a clean-up procedure. The gDNA may be extracted from a tissue or a powdered form thereof. The DNA sample may be treated with a proteinase (e.g., Proteinase K) to digest protein. The method may or may not include a phenol-chloroform extraction step to remove protein prior to further purification. In some embodiments, the DNA sample may be incubated in a lysis buffer and the lysate treated with a protease such as Proteinase K. Any cell lysis may be done with or without agitation, where the speed of agitation determines the median length of the resultant gDNA (see for example, FIG. 6). For example, a cell lysate (or tissue lysate) may be produced by agitating cells or ground tissue in lysis buffer, at a speed in the range of 300 rpm-2000 rpm at a temperature in the range of 37°C- 56°C. Agitation with 300 rpm -500 rpm can provide DNA in the Mb size range, whereas agitation at about 2000 rpm produces DNA that is 50 kb -250 kb.

The DNA sample may contain gDNA from any organism, e.g., plants, animals (e.g., reptiles, mammals, insects, worms, fish, etc.), tissue samples, bacteria, fungi (e.g., yeast), viruses, etc. In certain embodiments, the DNA purified by the present methods may be derived from a mammal, such as a human. In exemplary embodiments, the DNA sample may contain gDNA from a mammalian cell, such as, a human, mouse, rat, or monkey cell. The sample may be made from cultured cells or cells of a clinical sample, e.g., a tissue biopsy scrape or lavage, for example.

The eluted DNA may be analyzed using any suitable method. Table 4 shows the results of a spectrophotometric analysis. FIG. 6 shows a gel of sizes of DNA after agitation during lysis incubation at different speeds.

The eluted DNA may be sequenced using a long read single-molecule sequencing approach such as nanopore sequencing (e.g. as described in Soni, et al. Clin Chem 53: 1996-2001 2007, and developed by Oxford Nanopore Technologies) or Pacific Biosciences' fluorescent base-cleavage method (which currently has an read length of tens of kilobases, with some reads over 100 kb). Alternatively, the eluted DNA may be analyzed by other long-range genome mapping methods, such as the nanochannel array-based method (described by Lam, et al., Nat. Biotechnol. 2012 30: 771-776, as developed by BioNano Genomics, San Diego, CA).

### Compositions

A variety of compositions that are made during practice of the method are also provided. For example, the composition may contain one or more beads (e.g., 1-50 beads) where for example, the beads are spherical glass beads. The beads are required to have a minimum dimension of at least 200 µm (e.g., one or more beads have a minimum dimension of at least 1mm, such as in the range of 1 mm to 6 mm). Where the one or more beads are spherical, they have a diameter of at least 200 µm, such as a diameter of at least 1mm (e.g. 1 mm to 6 mm). The composition further includes a DNA preparation (e.g. DNA solution) comprising HMW DNA that has, for example, a median size of at least 35 kb or at least 50 kb; and a precipitant comprising alcohol, e.g., ethanol or isopropanol, with or without salt. In these embodiments, the components are in the same container, e.g., a 1.5 ml or 2 ml microcentrifuge tube, although any size tube may be utilized according to the volume of the sample. The DNA preparation or solution may be made from a cell or tissue lysate, e.g., bacterial or mammalian cells, for example. In alternative embodiments, a composition may comprise one or more beads (e.g., 1-50 beads in a 2 ml tube) that have a minimum dimension (e.g. diameter, if spherical) of at least 200 µm (e.g., one or more beads have a minimum dimension of at least 1mm, such as in the range of 1 mm to 6 mm). In these embodiments, the DNA is adhered to the surface of the beads. In these embodiments, the components may be within a bead containing tube with a top opening for receiving sample and beads, and an exit opening for releasing a DNA aqueous solution eluted from beads into a collection tube.

### Kits

Also provided by this disclosure are kits for practicing the subject method, as described above. In some embodiments, the kit may contain any one or more of the components listed above. For example, a kit may contain (a) one or more capture beads that have a minimum dimension of at least 200 µm (e.g., one or more beads that have a minimum dimension of at least 1mm, such as in the range of 1 mm to 6 mm), and (b) a bead retaining tube having one or more outlet openings having a size (e.g. diameter) that is smaller than the beads, and wherein the bead retaining tube is designed so that the one or more exit openings are not blocked by the one or more beads during use. Details of examples of beads and bead containing tubes that may be included in the kit are provided above. In some embodiments, a kit may additionally contain an outer tube (collection tube), wherein the bead retaining tube fits in the outer tube as illustrated in FIG. 1A and FIG. 4A. The outer tube may be a standard 1.5 ml or 2 ml microcentrifuge tube. In addition, a kit may further comprise one or more buffers such as a lysis buffer for lysing a biological sample containing cells, a protein removal solution, a wash buffer (e.g., an alcohol-containing wash buffer) for washing the DNA adhered beads, and/or an elution buffer for removing DNA from beads. The beads may be glass beads. The kit may also include a pestle and pestle tube for grinding up tissue or bacterial cells.

The various components of the kit may each be present in separate containers, or certain compatible components may be pre-combined into a single container, as desired. In addition to above-mentioned components, the subject kits may further include instructions for using the components of the kit to practice the subject methods, i.e., to instructions for sample analysis.

### EXAMPLES

Aspects of the present teachings can be further understood in light of the following examples, which should not be construed as limiting the scope of the present teachings in any way.

### Example 1: Preparation of deproteinized DNA samples from lysates

### (a) Preparation of HMW DNA from a suspension of cells.

Lymphocytes were obtained from whole blood (500 µl) as a pellet after centrifugation using standard techniques. The pellet was resuspended in phosphate buffered saline (PBS). Alternatively, cultured cells (HEK293) were obtained from cell culture.

The resuspended pellet containing lymphocytes or 5×10⁶ HEK293 cells in 150 microliters (µl) of PBS were then treated with 5 µl RNase A (20 mg/ml) and 10 µl of Proteinase K (20 mg/ml). 150 µl of Monarch^{®} DNA Blood lysis buffer (New England Biolabs, Ipswich, MA) was added and the cells incubated for 10 minutes at 56°C while shaken at 500 rpm -2000 rpm depending on the length of DNA required. For Mb DNA, it was found that 500 rpm was a maximum shaking speed. For 100 kb-250 kb DNA, 1400 rpm -2000 rpm was used (see FIG. 6). Since substantially all the protein was digested, a protein removal step was not required prior to the isolation of the HMW DNA (see the workflow in FIG. 1A).

### (b) Preparation of HMW DNA from solid tissue.

10 mg of tissue were prepared first by crushing in a micropestle (1.5 ml tube). Lysis buffer containing Proteinase K (10 µl of a solution of 20 mg/ml) plus 300 µl of buffer containing 50mM Tris, 2% SDS, 100 mM NaCl and 20 mM EDTA pH 88.25 was added so as to digest the protein in the sample. The sample was incubated at 56°C for 45 minutes in a shaker. The shaking speeds were as described in (a). 10 µl RNase A was added (20 mg/ml) and incubated 56°C for 10 minutes. Protein was separated from DNA by a chloroform/phenol step. The aqueous phase (400 µl) was recovered using a 1000 µl wide bore pipet tip and transferred to a 2 ml tube for precipitation of the HMW DNA (see FIG. 1B).

**Table 2: Examples of tissues with some alternative amounts, volumes of lysis buffer, Proteinase K and yield of DNA.**

| **Sample type** | **Amount (mg)** | **Tissue Lysis Buffer (µl)** | **Proteinase K (µl)** | **Expected yield (µg)** |
|---|---|---|---|---|
| Muscle/fibrous tissue | Up to 10 | 300 | 10 | 3-4 |
| | Up to 20 | 600 | 20 | 12-14 |
| Brain | Up to 10 | 300 | 10 | 3 |
| | Up to 20 | 600 | 20 | 6 |
| DNA-rich/soft organ tissue | Up to 5 | 300 | 10 | 5-15 |
| | Up to 10 | 600 | 20 | 10-30 |
| Rodent tail | Up to 10 | 300 | 10 | 10-15 |
| | Up to 20 | 600 | 20 | 20-30 |

For preparation of HMW DNA from solid tissue and cell suspension, a two-step lysis of cells was performed in which the cell membrane was disrupted releasing RNA while the nucleic remained intact. RNAse A was introduced to remove the RNA before lysis of the nuclei and release of gDNA. In this way, the RNA was removed before the viscosity of the lysate increased due to the release of the gDNA.

### (c) Preparation of HMW DNA from microbes

At least 2×10⁹ microbes were pelleted and resuspended in a lysis buffer containing 10 µl lysozyme (25 mg/ml), and 300 µl of tissue lysis buffer (50 mM Tris, 2% SDS, 100 mM NaCl and 20 mM EDTA pH 8.25) and incubated at 37°C at 1400 rpm-2000 rpm for 3 minutes -5 minutes. 20 µl of Proteinase K was then added and incubated at 56°C for a minimum of 30 minutes in a thermal mixer at maximum speed (1400 rpm -2000 rpm). 10 µl of RNase A was then added and incubated for 10 minutes at 56°C. Protein was separated from DNA by a chloroform/phenol step. The aqueous phase was recovered using a 1000 µl wide bore pipet tip and transferred to a 2 ml tube for concentration of the HMW DNA.

**Table 3: Preparation of deproteinized DNA samples from microbial lysates**

| **Number of bacterial cells** | **PBS (µl)** | **Tissue Lysis Buffer (µl)** | **Proteinase K (µl)** |
|---|---|---|---|
| Up to 2x10⁹ | 150 | 150 | 10 |
| Up to 5x10⁹ or unknown number | 300 | 300 | 20 |

### Example 2: Concentration of high molecular weight DNA

The following protocol describes an example of how HMW gDNA was isolated from a sample using capture beads. As shown in Table 4, different sized and shaped beads made of various materials to which DNA adsorbs can be used for this protocol while avoiding beads less than 200 µm that increase DNA shear and avoiding any form of filter with a pore size of less than 0.5 mm.

Samples described in FIG. 1A including lysates of blood samples or cultured cells or the non-protein phase of the deproteinated solid tissue or microbe sample were used in the following steps. The sample size was either 400 µl or 800 µl. Two DNA glass capture beads were added to each sample in a 2 ml tube.
(a) Binding of DNA to beads and removing contaminants by washing:
   275 µl of isopropanol (low input sample) or 275 µl isopropanol and 275 µl ethanol (high input sample) was then added to the sample to precipitate the DNA onto the capture beads.
(b) Washing the beads to remove DNA precipitant and lysate contaminants:
   800 µl of 70% ethanol was added to the sample. The wash buffer that contained a final concentration of 70% ethanol caused DNA to bind more tightly to the beads. The wash buffer was then decanted, and the beads were poured into an empty tube insert with an exit funnel (collar with exit opening) or porous mesh (pore size at least 0.5 mm) at the bottom of the tube. The collar with exit opening was designed so that the beads would not block the out flow of eluent. This was achieved by ridges on the collar of the exit funnel. The empty tube insert was placed into an empty 1.5 ml centrifuge tube. Examples of bead retaining tubes that can be used in the present method are shown in FIG. 2A-FIG.2B, FIG. 3A-FIG. 3B and FIG. 4A-FIG. 4B. The wash buffer was removed in a centrifugation step and was then repeated.
(c) Elution of DNA from beads in a concentrated solution:
   The dried beads containing the HMW DNA were poured into a 2 ml tube. Elution buffer (e.g., Tris EDTA) was added and incubated for 5 minutes -10 minutes at 56°C in a thermal mixer with agitation at lowest speed (300 rpm) or in heat block to permit the elution of DNA from the beads. The eluted DNA preparation (solution) and the beads were transferred to a bead retaining tube positioned in a 1.5 ml microfuge tube. The tube insert seated in the centrifuge tube was then centrifuged for 0.5 minutes at a max speed (>12,000 × g) to allow the gDNA to be collected in the centrifuge tube. The tube insert with glass beads was then discarded.

The samples contained HMW DNA and were stored at 4°C. The amount and purity of the DNA collected by this method was analyzed. Various borosilicate bead sizes, shapes and materials were tested, and the results are shown in Table 4. All beads functioned well as a binding matrix for gDNA providing similar yields for all types of glass beads. Beads of 1 mm and 2 mm diameter were collected in a flat bottom spin column from Corning, Inc. (Corning, NY) with openings of 0.5 mm max. All larger beads and the jewelry beads were collected in flat bottom spin columns from Corning, Inc. with openings of 1.5 mm max diameter.

**Table 4**

| **#** | **Bead type** | **No. of beads** | **Nucleic Acids (ng/µl)*** | **Yield (ug)** | **Background (A260)** | **Residue (%)** | **A260/A280** | **A260/A230** |
|---|---|---|---|---|---|---|---|---|
| 1 | 212-300 micron | 500 (9.1 mg) | 76.2 | 7.6 | 0 | 0.7 | 1.85 | 2.12 |
| 2 | 212-300 micron | 500 (9.1 mg) | 67.4 | 6.7 | 0 | 0.8 | 1.85 | 2.29 |
| 3 | 425-600 micron | 127 (18.4 mg) | 76.1 | 7.6 | 0 | 0.8 | 1.85 | 2.31 |
| 4 | 425-600 micron | 127 (18.4 mg) | 72.3 | 7.2 | 0 | 0.8 | 1.86 | 1.98 |
| 5 | 1mm | 32 (37.1 mg) | 63.5 | 6.4 | 0 | 0.9 | 1.87 | 2.40 |
| 6 | 1mm | 32 (37.1 mg) | 77.5 | 7.8 | 0 | 1.3 | 1.87 | 2.48 |
| 7 | 2 mm | 8 | 75.7 | 7.6 | 0 | 0.8 | 1.88 | 2.41 |
| 8 | 2 mm | 8 | 79.0 | 7.9 | 0 | 0.9 | 1.86 | 2.43 |
| 9 | 3 mm | 4 | 65.0 | 6.5 | 0 | 3.6 | 1.85 | 2.91 |
| 10 | 3 mm | 4 | 69.5 | 7.0 | 0 | 2.1 | 1.89 | 2.61 |
| 11 | 4 mm | 2 | 78.6 | 7.9 | 0 | 1.3 | 1.87 | 2.51 |
| 12 | 4 mm | 2 | 80.0 | 8.0 | 0 | 0.6 | 1.90 | 2.39 |
| 13 | 5 mm | 1 | 73.1 | 7.3 | 0 | 0.7 | 1.83 | 2.35 |
| 14 | 5 mm | 1 | 78.9 | 7.9 | 0 | 0.7 | 1.88 | 2.33 |
| 15 | 6 mm | 1 | 81.6 | 8.2 | 0 | 1.0 | 1.89 | 2.26 |
| 16 | 6 mm | 1 | 76.5 | 7.7 | 0 | 0.9 | 1.90 | 2.27 |
| 17 | rectangular black glass bead | 1 | 75.6 | 7.6 | 0.25 | 0.6 | 1.86 | 2.55 |
| 18 | long disc shaped green glass bead | 1 | 80.1 | 8.0 | 0 | 0.8 | 1.88 | 2.40 |
| 19 | 8-shaped long green glass bead | 1 | 71.2 | 7.1 | 0.71 | 7.5 | 1.74 | 4.02 |
| 20 | double hexagonal shaped violet glass bead | 1 | 78.8 | 7.9 | 0.35 | 1.3 | 1.85 | 2.40 |
| 21 | blue glass bead | 1 | 80.9 | 8.1 | 0 | 1.1 | 1.89 | 2.28 |
| 22 | double hexagonal shaped small orange glass bead | 1 | 77.4 | 7.7 | 0 | 1.4 | 1.90 | 2.32 |
| 23 | 8-shaped orange with eyes glass bead | 1 | 83.1 | 8.3 | 0.85 | 1.2 | 1.81 | 2.25 |
| 24 | cylinder shaped glass bead | 1 | 82.6 | 8.3 | 0.93 | 2.6 | 1.79 | 2.31 |

| | | | | | | | | |
|---|---|---|---|---|---|---|---|---|
| • The amount of DNA was determined from 2 µl samples using Trinean DropSense^{®} 16 spectrophotometer (Unchained Labs, Woburn, MA) | | | | | | | | |

As shown in the table above, all large size beads worked well for isolating HMW gDNA from cultured cells. Beads ranging from 1 mm-6 mm were identified as being most practical, since they could be placed into in bead retaining tubes with large sieve openings, which allows for a convenient separation of the beads and the gDNA containing eluate by centrifugation in a microcentrifuge without shearing the gDNA. In addition, in these bead retaining tubes traces of ethanol containing wash buffer can be removed from the gDNA on the glass beads by spinning them very briefly in a benchtop mini centrifuge.

### Example 3: Measuring and Analyzing HMW DNA samples

Purified HMW gDNA is often viscous, tends to clump and is challenging to handle and transfer volumes with accuracy. For testing the purity of the DNA samples, the samples were homogenized by using a wide bore pipette tip or needle or by a short vortex. HMW DNA for Oxford nanopore sequencing is preferably 50 kb-250 Kb in length for optimal sequencing results. To obtain DNA of this size, an agitation of 2000 rpm during lysis provided optimal results. Additionally, 10-20 passes through a 26 gauge needle was used for tissue and bacteria samples that were isolated starting with homogenization with the microtube pestle (tissue) or not homogenized (bacteria).

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

### CLAUSES

1. A kit comprising: (a) one or more beads that have a smallest dimension of at least 200 µm; and (b) a bead retaining tube having an opening for receiving a DNA preparation and one or more exit openings, wherein each exit opening has a size that is smaller than the smallest dimension of the one or more beads, and wherein the bead retaining tube comprises: (i) at least one ridge for supporting the one or more beads above the one or more exit openings so that the one or more exit openings remain unblocked by the one or more beads; or (ii) a series of exit openings arranged in a grid.
2. The kit of clause 1, wherein the one or more beads have a smallest dimension of at least 1mm, such as in the range of 1 mm - 6 mm.
3. The kit according to clause 1 or 2, wherein the one or more beads are formed of rough or smooth material having a surface charge; and/or wherein the one or more beads are formed from glass or are ceramic beads.
4. The kit according to any preceding clause, wherein the one or more beads are spherical.
5. The kit according to clause 4, wherein the one or more beads has a diameter of at least 200 µm, optionally at least 1mm, such as in the range of 1 mm - 6 mm.
6. The kit according to clause 4 or 5, wherein the one or more exit openings of the bead retaining tube are circular and have a diameter that is smaller than the diameter of the beads; optionally wherein the one or more spherical beads each have a diameter of at least 1mm and the one or more circular exit openings have a diameter that is at least 0.5 mm but less than 1mm.
7. The kit according to any preceding clause, comprising 1-50 spherical beads each with a diameter in the range of 1 mm - 6 mm, wherein the number of beads in the kit is inversely proportional to the diameter of the beads; and wherein the bead retaining tube has a volume large enough to contain the beads.
8. The kit according to any preceding clause, wherein the kit further comprises an outer tube, wherein the bead retaining tube fits into the outer tube; optionally wherein the outer tube has a volume of 1 ml - 2 ml.
9. The kit according to any preceding clause, wherein the kit further comprises: an elution buffer suitable for removing DNA adhered to the beads; a wash buffer to remove contaminants from the DNA adhered to the beads; and/or a lysis buffer for lysing cells from a biological fluid or tissue.
10. A composition comprising: (a) one or more beads having a smallest dimension of at least 200 µm; (b) a DNA preparation comprising DNA that has a median size of at least 35 kb; and (c) a DNA precipitant.
11. The composition according to clause 10, wherein: the one or more beads have a smallest dimension of at least 1mm, such as a smallest dimension in the range of 1 mm - 6 mm; or the one or more beads are spherical and have a diameter of at least 200 µm, such as at least 1mm, such as in the range of 1 mm - 6 mm; optionally wherein the one or more spherical beads are 1-50 spherical beads and/or wherein the one or more spherical beads are spherical glass beads.
12. The composition according to clause 10 or 11, wherein the DNA preparation is obtained from a cell lysate, such as wherein the DNA preparation is the product of a cleanup reaction from a cell lysate; or wherein the DNA preparation is a cell lysate or a tissue lysate.
13. The composition according to any of clauses 10-12, wherein the DNA precipitant is selected from an alcohol, a chaotropic salt and a crowding agent.
14. The composition according to any of clauses 10-13, wherein the one or more beads, the DNA preparation, and the DNA precipitant are contained in a bead retaining tube, wherein the DNA adheres to the beads.
15. A method comprising: (a) providing a composition comprising: (i) one or more beads having a smallest dimension of at least 200 µm; (ii) a DNA preparation comprising a high molecular weight (HMW) DNA; and (iii) a DNA precipitant; (b) incubating the composition to adhere the DNA to the one or more beads; (c) removing unbound material from the one or more beads; (d) washing the one or more beads with a wash buffer; (e) releasing the DNA from the one or more beads into an elution buffer; and (f) separating the released DNA in the eluate from the one or more beads.
16. The method according to clause 15, wherein: the one or more beads have a smallest dimension of at least 1mm, such as in the range of 1 mm - 6 mm; or the one or more beads are spherical beads having a diameter of at least 200 µm, such as at least 1mm, such as in the range of 1 mm - 6 mm; optionally wherein the one or more spherical beads are 1-50 spherical beads, and/or wherein the one or more beads are spherical glass beads.
17. The method according to clause 15 or 16, wherein the DNA preparation is a complex mixture comprising DNA and macromolecules, such as a cell lysate; optionally wherein the DNA preparation is a product of lysing cells to produce a lysate, centrifuging the lysate, and using the supernatant or the lysate as the DNA preparation; optionally wherein the cell lysate is produced by agitating cells in a lysis buffer at 300 rpm-2000 rpm at a temperature in the range of 37°C- 56°C, wherein the speed of agitation determines the median length of the DNA used in step (a).
18. The method according to any of clauses 15-17, wherein the high molecular weight (HMW) DNA has a size of at least 35 kb, such as at least 50 kb; and/or wherein the released DNA is a high molecular weight (HMW) DNA having a median size of at least 25 kb or 35 kb.
19. The method according to any of clauses 15-18, wherein the DNA precipitant of (a)(iii) comprises an alcohol such as ethanol, isopropanol, a crowding agent and/or chaotropic salts.
20. The method according to any of clauses 15-19, wherein step (c) is done by pouring or pipetting.
21. The method according to any of clauses 15-20, wherein washing step (d) is done using an alcohol-containing wash buffer, and optionally comprises pouring the bead(s) into a bead retaining tube, and spinning the bead retaining tube; and/or wherein washing step (d) comprises removing residual non-DNA material.
22. The method according to any of clauses 15-20, wherein steps (e) and (f) are done by incubating the washed one or more beads in an elution buffer to dissolve the DNA in a bead retaining tube, spinning the bead retaining tube, and collecting the eluate containing the DNA in a collection tube.
23. The method according to clause 21 or 22, wherein the bead retaining tube has one or more outlet openings, wherein each outlet opening has a size that is less than the smallest dimension of the one or more beads, and wherein the bead retaining tube comprises: (i) at least one ridge for supporting the one or more beads above the one or more exit openings so that the one or more exit openings remain unblocked by the one or more beads; or (ii) a series of exit openings arranged in a grid; optionally wherein the one or more beads are one or more spherical beads each having a diameter of at least 1mm and the one or more outlet openings are one or more circular openings having a diameter that is at least 0.5 mm but less than 1mm.

## Claims

1. A kit comprising:
(a) one or more beads that have a smallest dimension of at least 200 µm; and
(b) a bead retaining tube having an opening for receiving a DNA preparation and one or more exit openings, wherein each exit opening has a size that is smaller than the smallest dimension of the one or more beads, and wherein the bead retaining tube comprises:
(i) at least one ridge for supporting the one or more beads above the one or more exit openings so that the one or more exit openings remain unblocked by the one or more beads; or
(ii) a series of exit openings arranged in a grid.

2. The kit according to claim 1, wherein the one or more beads are formed of rough or smooth material having a surface charge; and/or wherein the one or more beads are formed from glass or are ceramic beads.

3. The kit of claim 1 or 2, wherein the one or more beads have a smallest dimension of at least 1mm, such as in the range of 1 mm - 6 mm.

4. The kit according to claim 1 or claim 2, wherein:
the one or more beads are spherical; optionally wherein the one or more beads has a diameter of at least 200 µm, optionally at least 1mm, such as in the range of 1 mm - 6 mm; and/or
the one or more exit openings of the bead retaining tube are circular and have a diameter that is smaller than the diameter of the beads; optionally wherein the one or more spherical beads each have a diameter of at least 1mm and the one or more circular exit openings have a diameter that is at least 0.5 mm but less than 1mm.

5. The kit according to any preceding claim, comprising 1-50 spherical beads each with a diameter in the range of 1 mm - 6 mm, wherein the number of beads in the kit is inversely proportional to the diameter of the beads; and wherein the bead retaining tube has a volume large enough to contain the beads.

6. The kit according to any preceding claim, wherein the kit further comprises:
an outer tube, wherein the bead retaining tube fits into the outer tube; optionally wherein the outer tube has a volume of 1 ml - 2 ml; and/or
an elution buffer suitable for removing DNA adhered to the beads; a wash buffer to remove contaminants from the DNA adhered to the beads; and/or a lysis buffer for lysing cells from a biological fluid or tissue.

7. A composition comprising:
(a) one or more beads having a smallest dimension of at least 200 µm;
(b) a DNA preparation comprising DNA that has a median size of at least 35 kb; and
(c) a DNA precipitant, such as an alcohol, a chaotropic salt, or a crowding agent.

8. The composition according to claim 7, wherein the DNA preparation is obtained from a cell lysate, such as wherein the DNA preparation is the product of a cleanup reaction from a cell lysate; or wherein the DNA preparation is a cell lysate or a tissue lysate.

9. The composition according to claim 7 or claim 8, wherein the one or more beads, the DNA preparation, and the DNA precipitant are contained in a bead retaining tube, wherein the DNA adheres to the beads.

10. A method comprising:
(a) providing a composition comprising:
(i) one or more beads having a smallest dimension of at least 200 µm;
(ii) a DNA preparation comprising a high molecular weight (HMW) DNA; and
(iii) a DNA precipitant, such as an alcohol, a crowding agent, and/or a chaotropic salt;
(b) incubating the composition to adhere the DNA to the one or more beads;
(c) removing unbound material from the one or more beads, such as by pouring or pipetting;
(d) washing the one or more beads with a wash buffer;
(e) releasing the DNA from the one or more beads into an elution buffer; and
(f) separating the released DNA in the eluate from the one or more beads.

11. The method according to claim 10, wherein the DNA preparation is a complex mixture comprising DNA and macromolecules, such as a cell lysate;
optionally wherein the DNA preparation is a product of lysing cells to produce a lysate, centrifuging the lysate, and using the supernatant or the lysate as the DNA preparation;
optionally wherein the cell lysate is produced by agitating cells in a lysis buffer at 300 rpm-2000 rpm at a temperature in the range of 37°C- 56°C, wherein the speed of agitation determines the median length of the DNA used in step (a).

12. The method according to claim 10 or 11, wherein the high molecular weight (HMW) DNA has a size of at least 35 kb, such as at least 50 kb; and/or wherein the released DNA is a high molecular weight (HMW) DNA having a median size of at least 25 kb or 35 kb.

13. The method according to any of claims 10-12, wherein:
washing step (d) is done using an alcohol-containing wash buffer, and optionally comprises pouring the bead(s) into a bead retaining tube, and spinning the bead retaining tube; and/or
wherein washing step (d) comprises removing residual non-DNA material; or steps (e) and (f) are done by incubating the washed one or more beads in an elution buffer to dissolve the DNA in a bead retaining tube, spinning the bead retaining tube, and collecting the eluate containing the DNA in a collection tube.

14. The method according to claim 13, wherein the bead retaining tube has one or more outlet openings, wherein each outlet opening has a size that is less than the smallest dimension of the one or more beads, and wherein the bead retaining tube comprises:
(i) at least one ridge for supporting the one or more beads above the one or more exit openings so that the one or more exit openings remain unblocked by the one or more beads; or
(ii) a series of exit openings arranged in a grid;
optionally wherein the one or more beads are one or more spherical beads each having a diameter of at least 1mm and the one or more outlet openings are one or more circular openings having a diameter that is at least 0.5 mm but less than 1mm.

15. The composition according to any of claims 7-9 or the method according to any of claims 10-14, wherein:
the one or more beads have a smallest dimension of at least 1mm, such as in the range of 1 mm - 6 mm; or
the one or more beads are spherical beads having a diameter of at least 200 µm, such as at least 1mm, such as in the range of 1 mm - 6 mm; optionally wherein the one or more spherical beads are 1-50 spherical beads, and/or wherein the one or more beads are spherical glass beads.
